# EUROPEAN PATENT APPLICATION

(11) **EP 3 553 052 A1**
(43) Date of publication of application: **16.10.2019**
(21) Application number: 19167010.8
(22) Date of filing: 03.04.2019
(51) Int. Cl.: C07D 249/12, C07C 235/64, A61P 17/02, A61P 17/06, A61P 35/00, A61K 31/4196

(54) **5-OXO-4,5-DIHYDRO-1H-1,2,4-TRIAZOL DERIVATIVES FOR THE TREATMENT OF CANCER**

(30) Priority: 10.04.2018 EP 18166556
(71) Applicant: Bayer AG, 51373 Leverkusen (DE)
(72) Inventor: Gradl, Stefan, Nikolaus, 13156 Berlin (DE); Niehues, Michael, 12159 Berlin (DE); Halfbrodt, Wolfgang, 13505 Berlin (DE)
(74) Representative: BIP Patents

(57) **Abstract**

The present invention provides Compound (I), 2-butoxy-N-(2-chloro-6-fluorophenyl)-4-[3-(2-chlorophenyl)-5-oxo-4,5-dihydro-1H-1,2,4-triazol-1-yl]-5-fluorobenzamide and Compound (II), N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, (II): 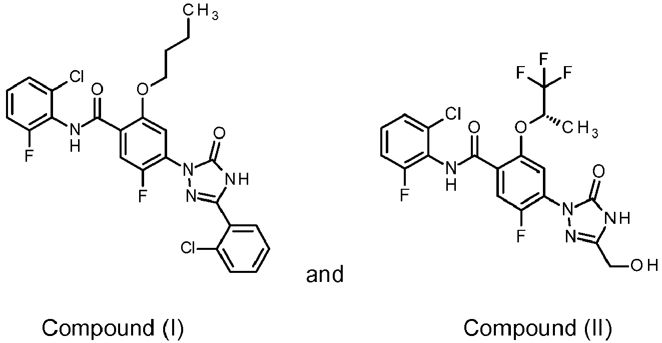 and stereoisomers, tautomers, N-oxides, hydrates, solvates, and salts thereof, and mixtures of same, methods for the preparation of Compound (I) and Compound (II) and their use for the treatment of hyperproliferative diseases, particularly cancer.

## Description

The present invention provides 5-oxo-4,5-dihydro-1H-1,2,4-triazol compounds as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of cancer disorders, as a sole agent or in combination with other active ingredients.

### BACKGROUND

The present invention provides 5-oxo-4,5-dihydro-1H-1,2,4-triazol compounds which inhibit Dihydroorotate Dehydrogenase (DHODH).

Acute myeloid leukemia (AML) is the most common acute leukemia in humans with a 5 year survival of only about 30%. AML is a malignancy of the myeloid line of blood cells. The incidence rates and chances of cure are highly age dependent. The chemotherapy standard of care for AML has not changed significantly over the last decades highlighting the need for novel therapies. A major hallmark of AML is differentiation arrest of the leukemic cells at early stages of cellular differentiation. The potential of leukemic differentiation therapy can be seen with the success of ATRA or arsenic trioxide inducing differentiation in acute promyelocytic leukemia (APL). Around 10% of AML belong to the APL subtype where leukemic cells are harbouring a chromosomal translocation resulting in fusions of oncoproteins involving the retinoic acid receptor. While treatment with ATRA or arsenic trioxide leads to a dramatic increase of patient survival, with overall survival rates of over 70%, unfortunately a comparable differentiation therapy for the non-APL AMLs is lacking (Management of acute promyelocytic leukemia: recommendations from an expert panel on behalf of the European LeukemiaNet, Sanz M.A. et al, Blood 2009, 113(9), 1875-1891). Therefore new therapies inducing differentiation of AML cells are of high interest and medical need.

### Dihydroorotate Dehydrogenase (DHODH)

DHODH is located in the mitochondria and the enzyme responsible for the 4th and rate limiting step in de novo pyrimidine synthesis converting dihydroorotate to orotate (Dihydroorotatubiquinone oxidoreductase links mitochondria in the biosynthesis of pyrimidine nucleotides, Löffler M. et al, Molecular and Cellular Biochemistry 1997, 174, 125-129).

As pyrimdine production is essential for DNA and RNA synthesis DHODH is highly important for cellular proliferation. The enzyme is considered an attractive drug target for cancer, immunological, parasitic and viral diseases and DHODH small molecule inhibitors like Leflunomide/Teriflunomide and Brequinar have been approved for clinical use in Rheumatoid Arthritis and Multiple Sclerosis. Additionally, preclinical studies indicate that DHODH inhibitors may be useful for the treatment of haematological cancer indications, for the treatment of solid tumors (e.g., neuroblastoma, melanoma, colon, breast and lung tumors), for the treatment of parasitic diseases (e.g., malaria), and for viral disease therapy.

### STATE OF THE ART

US 6444613 B1 relates to the field of defoliants, in particular thidiazuron-comprising mixtures, and their use in crops of cotton. These mixtures comprise among others 2,4,5-trisubstituted 1,2,4-triazolone compounds as herbicides, which inhibit the enzyme protoporphyrinogen-(IX) oxidase (PPO inhibitors).

WO199802422 describes substituted aromatic carbonyl compounds, among others 2,4,5-trisubstituted 1,2,4-triazolone compounds, as herbicides.

From CN106543139 some triazolone compounds are known as agrochemicals.

US 2016/0251341 A1 describes triazole compounds as serine protease inhibitors useful for the inhibition of thrombin and/or kallikrein.

WO2010/077686 A1 describes sirtuin-modulating compounds, e.g. isoindolinone and related compounds, and methods of use thereof. The sirtuin-modulating compounds may be used for increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing as well as diseases or disorders that would benefit from increased mitochondrial activity.

WO 2013/186692 A1 describes triazolone compounds as mPGES-1 inhibitors, useful in the treatment of pain and/or inflammation from a variety of diseases or conditions, such as asthma, osteoarthritis, rheumatoid arthritis, acute or chronic pain and neurodegenerative diseases.

However, the state of the art does not describe the specific 2,4,5-trisubstituted 1,2,4-triazolone compound (I) or (II) of the present invention as described and defined herein.

### DETAILED DESCRIPTION

It has now been found, and this constitutes the basis of the present invention, that Compound (I), 2-butoxy-N-(2-chloro-6-fluorophenyl)-4-[3-(2-chlorophenyl)-5-oxo-4,5-dihydro-1H-1,2,4-triazol-1-yl]-5-fluorobenzamide and Compound (II), N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, have surprising and advantageous properties.

In particular, the compounds have surprisingly been found to inhibit dihydroorotate dehydrogenase and may therefore be used for the treatment of hyperproliferative diseases, particularly cancer.

In accordance with a first aspect, the present invention provides Compound (I) and Compound (II): 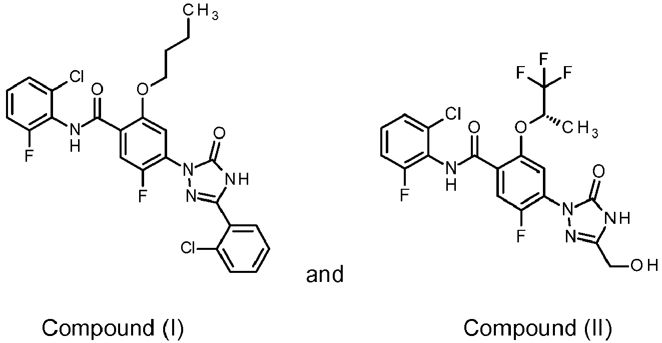 and stereoisomers, tautomers, N-oxides, hydrates, solvates, and salts thereof, and mixtures of same.

In accordance with a further aspect, the present invention provides methods for the preparation of Compound (I) and Compound (II) and their use for the treatment of hyperproliferative diseases, particularly cancer.

### DEFINITIONS

Unless otherwise noted, generally acceptable polar aprotic solvents may be, for example, ethers such as diethyl ether, tert-butyl methyl ether, tetrahydrofuran (THF), 1,4-dioxane or 1,2-dimethoxyethane, hydrocarbons such as benzene, toluene, xylene, hexane or cyclohexane, halogenated hydrocarbons such as dichloromethane, trichloromethane, carbon tetrachloride, 1,2-dichloroethane, trichloroethylene or chlorobenzene, or other solvents such as acetone, acetonitrile, ethyl acetate, pyridine, dimethylsulfoxide (DMSO), N,N-dimethyl-formamide (DMF) or N methylpyrrolidinone (NMP). It is also possible to use mixtures of these solvents.

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "usual work-up" or "isolation" means isolating the reaction product from the reaction mixture by methods of a person skilled in the art comprising but not limited to steps like e.g. separating organic phase from aqueous phase, drying the organic phase with alkali sulfates such as sodium sulfate or potassium sulfate, or magnesium sulfate or calcium sulfate, filtering and evaporating the solvent to obtain crude product, dissolving again the crude product and cooling down to crystallize. If these steps are expressly mentioned, the method may be part of the invention.

The term "isolation" of a reaction product includes the usual work-up as well as optionally destillation, crystallization and chromatography.

The term "purification" includes destillation, crystallization, recrystallization and chromatography.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

The term "room temperature" is meant to be within a range of 20"-24°C.

It is possible for the Compounds (I) and (I) to exist as isotopic variants. The invention therefore includes one or more isotopic variant(s) of compound (I) or (II), particularly deuterium-containing compound (I) or (II).

The term "Isotopic variant" of a compound or a reagent is defined as a compound exhibiting an unnatural proportion of one or more of the isotopes that constitute such a compound.
mixture.

The term "Isotopic variant of compound (I) or (II) is defined as compound (I) or (II) exhibiting an unnatural proportion of one or more of the isotopes that constitute such a compound.

The expression "unnatural proportion" means a proportion of such isotope which is higher than its natural abundance. The natural abundances of isotopes to be applied in this context are described in "Isotopic Compositions of the Elements 1997", Pure Appl. Chem., 70(1), 217-235, 1998.

Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I, respectively.

With respect to the treatment and/or prophylaxis of the disorders specified herein the isotopic variant(s) of the compound (I) or (II)preferably contain deuterium ("deuterium-containing compound (I) or (II)"). Isotopic variants of compound (I) or (II)in which one or more radioactive isotopes, such as ³H or ¹⁴C, are incorporated are useful e.g. in drug and/or substrate tissue distribution studies. These isotopes are particularly preferred for the ease of their incorporation and detectability. Positron emitting isotopes such as ¹⁸F or ¹¹C may be incorporated into compound (I) or (II). These isotopic variants of compound (I) or (II) are useful for in vivo imaging applications. Deuterium-containing and ¹³C-containing compound (I) or (II) can be used in mass spectrometry analyses in the context of preclinical or clinical studies.

Isotopic variants of compound (I) or (II)can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from D₂O can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.

The term "deuterium-containing compound (I) or (II)" is defined as compound (I) or (II), in which one or more hydrogen atom(s) is/are replaced by one or more deuterium atom(s) and in which the abundance of deuterium at each deuterated position of compound (I) or (II) is higher than the natural abundance of deuterium, which is about 0.015%. Particularly, in a deuterium-containing compound (I) or (II)the abundance of deuterium at each deuterated position of the compound (I) or (II) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into compound (I) or (II) may alter the physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound (I) or (II). In some cases deuterium substitution reduces or eliminates the formation of an undesired or toxic metabolite and enhances the formation of a desired metabolite (e.g. Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (e.g. Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

Compound (I) or (II) may have multiple potential sites of attack for metabolism. To optimize the above-described effects on physicochemical properties and metabolic profile, deuterium-containing compound (I) or (II) having a certain pattern of one or more deuterium-hydrogen exchange(s) can be selected. Particularly, the deuterium atom(s) of deuterium-containing compound (I) or (II) is/are attached to a carbon atom and/or is/are located at those positions of compound (I) or (II), which are sites of attack for metabolizing enzymes such as e.g. cytochrome P₄₅₀.

In another embodiment the present invention concerns a deuterium-containing Compound (I), comprising one or more groups selected from CD₃, OCD₃, benzylic CD₂.

Where the plural form of the word compounds, salts, polymorphs, hydrates, solvates and the like, is used herein, this is taken to mean also a single compound, salt, polymorph, isomer, hydrate, solvate or the like, especially is includes Compound (I) and Compound (II).

By "stable compound' or "stable structure" is meant a compound that is sufficiently robust to survive isolation to a useful degree of purity from a reaction mixture, and formulation into an efficacious therapeutic agent.

Should there occur optical isomers those can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., HPLC columns using a chiral phase), with or without conventional derivatisation, optimally chosen to maximise the separation of the enantiomers. Suitable HPLC columns using a chiral phase are commercially available, such as those manufactured by Daicel, e.g., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.

In order to distinguish different types of isomers from each other reference is made to IUPAC Rules Section E (Pure Appl Chem 45, 11-30, 1976).

Further, it is possible for the compounds of the present invention to exist as tautomers. For example, any compound of the present invention which contains an imidazopyridine moiety as a heteroaryl group for example can exist as a different tautomers, or even a mixture in any amount of the two tautomers, for example:

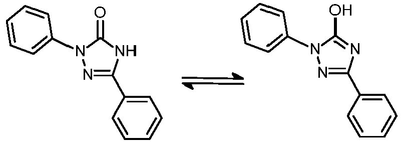

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also provides useful forms of the compounds of the present invention, such as metabolites, hydrates, solvates, prodrugs, salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, e.g. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N*-methylpiperidine, *N*-methyl-glucamine, *N*,*N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(*n*-butyl)ammonium, *N*-benzyl-*N*,*N*,*N-*trimethylammonium, choline or benzalkonium.

Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

Furthermore, the present invention includes all possible crystalline forms, or polymorphs, of the compounds of the present invention, either as single polymorph, or as a mixture of more than one polymorph, in any ratio.

Moreover, the present invention also includes prodrugs of the compounds according to the invention. The term "prodrugs" here designates compounds which themselves can be biologically active or inactive, but are converted (for example metabolically or hydrolytically)

Further embodiments of the first aspect of the present invention:
In a particular further embodiment of the first aspect, the present invention provides combinations of two or more of the above mentioned embodiments under the heading "further embodiments of the first aspect of the present invention".

The present invention provides any sub-combination within any embodiment or aspect of the present invention of compound (I) or (II), *supra.*

The present invention provides any sub-combination within any embodiment or aspect of the present invention of intermediate compound (VII).

The present invention provides compound (I) or (II) which are disclosed in the Example Section of this text, *infra.*

Compounds (I) or (II) can be prepared according to the following schemes 1, 2 and 3. The schemes and procedures described below illustrate synthetic routes compound (I) and (II) of the invention and are not intended to be limiting. It is clear to the person skilled in the art that the order of transformations as exemplified in schemes 1, 2 and 3 can be modified in various ways. The order of transformations exemplified in these schemes is therefore not intended to be limiting. In addition, interconversion of any of the substituents, R¹, R², R³, R⁴, R⁵ or R⁶ can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

Two routes for the preparation of Compounds (I) and (II) are described in Schemes 1, and 2.

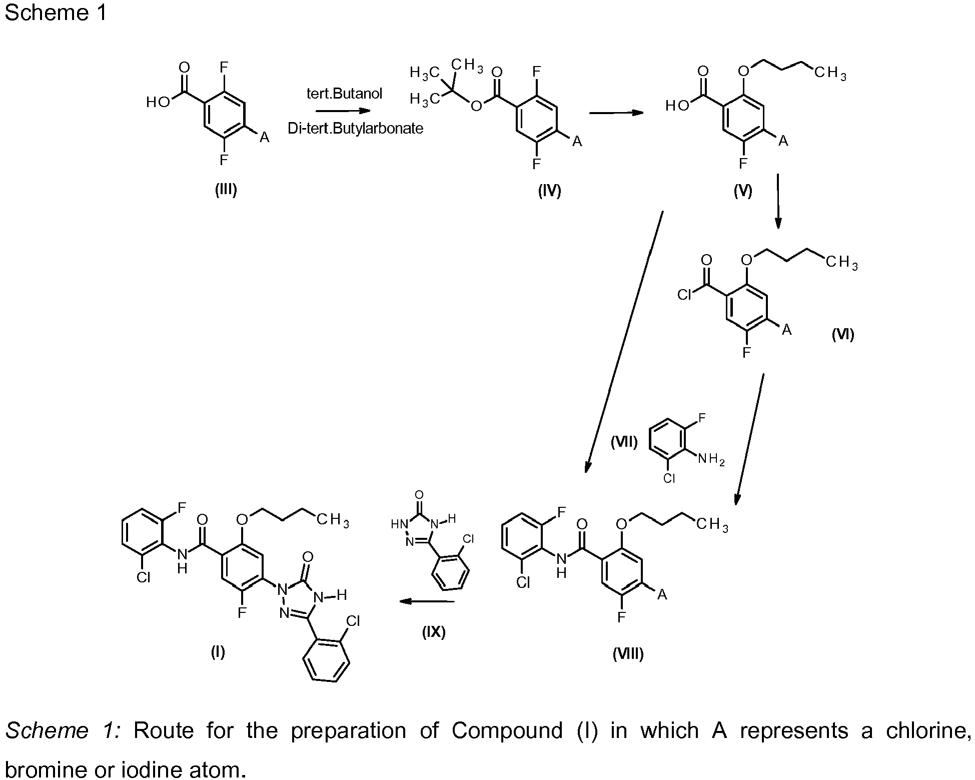

Compounds (VII) and (IX) are either commercially available or can be prepared according to procedures available from the public domain, as understandable to the person skilled in the art. Specific examples are described in the Experimental Section.

### General description of scheme 1

### Compound (III) to Compound (IV):

4-Halo-2,5-difluorobenzoic acid, preferably 4-Bromo-2,5-difluorobenzoic acid, is allowed to react with di-tert.butyl dicarbonate in an alcohol, perferably in tert. butanol optionally under addition of a catalyst as coupling reagent such as e.g. 4-dimethylaminopyridine (DMAP), DCC, EDC, HATU, preferably DMAP. After sufficient time the reaction mixture was concentrated and a usual work-up procedure was performed. The resulting compound (IV) could be used for the next step without further purification.

### Compound (IV) to Compound (V):

The alcohol intended to become the phenolic ether, preferably butanol, is dissolved in a polar solvent, preferably tetrahydrofurane (THF), cooled and reacted with 4-halo-2,5-difluorobenzoate, preferably the 4-bromo-2,5-difluorobenzoate (IV) under addition of a basic catalyst, sodium-bis(trimethylsilyl)amide, potassium-bis(trimethylsilyl)amide (KHMDS), lithium-tetramethylpiperidide (LiTMP), Lithiumdiisoprpylamine, N,N-diisopropylethylamine (DIPEA, also called Hünig's Base), 2,6-di-tert-butylpyridine, phosphazene bases, such as t-Bu-P4, preferably potassium bis(trimethylsilyl)amide. After a suitable reaction time the mixture is diluted with dichloromethane (DCM) and a usual work-up process was performed. The 4-halo-2-butoxy-5-fluorobenzoate. Preferably the 4-bromo-2-butoxy-5-fluorobenzoate was isolated and directly used for the next reaction step which was the cleavage of the tert.butylester by reaction of the benzoate in a polar protic solvent, e.g. in a dioxane/water mixture with an alkalihydroxide or an alkaline erth metal hydroxide such as e.g. lithiumhydroxide, sodiumhydroxide, potassiumhydroxide, calciumhydroxide or magesiumhydroxide, preferably lithiumhydroxide. After usual work-up the 4-halo-2-butoxy-5-fluorobenzoic acid, preferably the 4-bromo-2-butoxy-5-fluorobenzoic acid (V) was obtained.

### Compound (V) to Compound (VI) to Compound (VIII):

Compound (V) was diluted in a polar solvent and allowed to react with a acid chloride building reagent, such as e.g. thionyl chloride, oxalyl chloride, optionally under catalysis of dimethylformamide (DMF). After a suitable period of time, the amine, preferably 2-chloro-6-fluoroaniline (VII), was added together with a suitable base, such as e.g. triethylamine. After sufficient reaction time the mixture was concentrated under reduced pressure, the residue was dissolved in an alcohol, such as e.g. methanol or ethanol, preferably in ethanol and the product (VII) obtained as solid after addition of water and isolation by filtration.

### Compound (V) to Compound (VIII):

Interconversion of compound (V) to compound (VIII) could alternatively be achieved using suitable amide coupling reagents as known by the preson skilled in the art.

### Compound (VIII) to Compound (I):

The 4-halo-2-butoxy-N-(2-chloro-6-fluorophenyl)-5-fluorobenzamide, preferably the 4-bromo-2-butoxy-N-(2-chloro-6-fluorophenyl)-5-fluorobenzamide was allowed to react with 5-(2-chlorophenyl)-2,4-dihydro-3H-1,2,4-triazol-3-one (IX) (which also may be made following analogously the instructions to US 5, 436, 252) under addition of cesium carbonate, powdered molecular sieve (3Å), in a polar solvent, preferably dioxane, under palladium catalysis of [(2-Di-tert-butylphosphino-3,6-dimethoxy-2',4',6'-triisopropyl-1,1'-biphenyl)-2-(2'-amino-1,1'-biphenyl)]palladium(II) methanesulfonate (tBuBrettPhos-G3-Pd) and 2-(Di-tert-butylphosphino)-2',4',6'- triisopropyl-3,6-dimethoxy-1,1'-biphenyl (tBuBrettPhos) and oxygen free conditions. After some reaction time additional catalyst was added and the mixrue heated to about 70°C. When reaction was finaized the suspension was filtered, washed and concentrated. After purification, preferably by chromatography, Compound (I) was obtained.

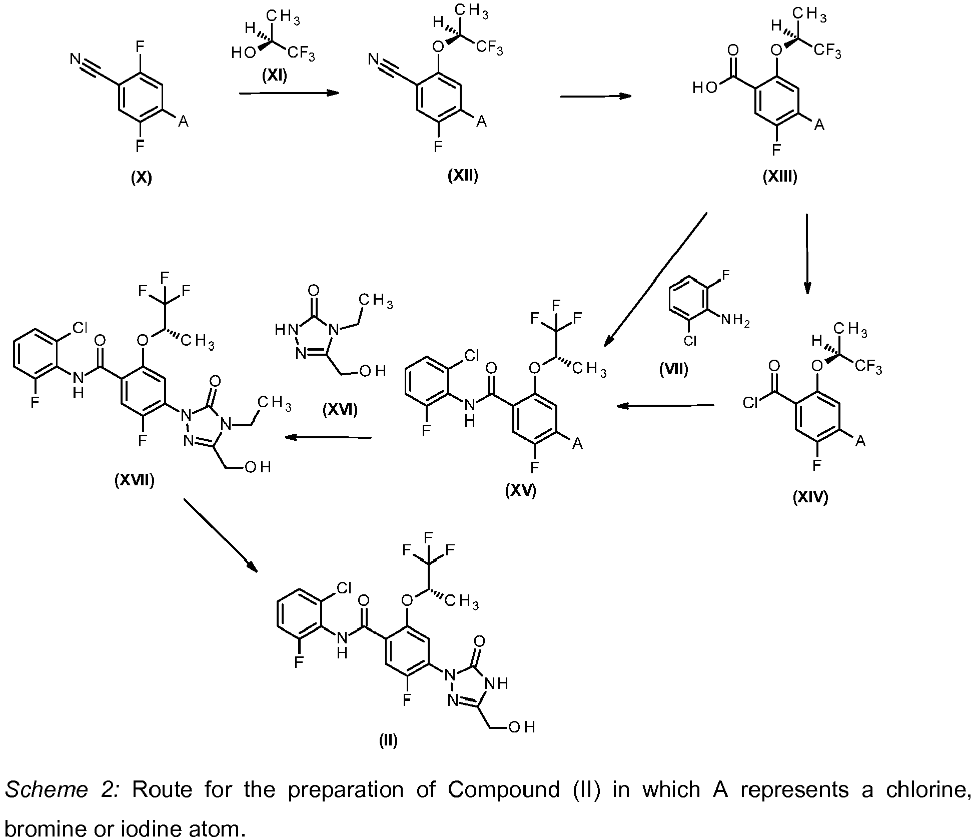

Compounds of formulae (X), (XVI), and (VII) are either commercially available or can be prepared according to procedures available from the public domain, as understandable to the person skilled in the art. Specific examples are described in the Experimental Section.

### Compound (X) to Compound (XII):

Nitriles of formula (XII) can be prepared from nitriles of formula (X) and with alcohols of formula (XI) according to procedures available from the public domain, as understandable to the person skilled in the art, sodium hydride in DMF may be used, alternatively, for alcohols of sufficiently high acidity, such as e.g. trifluoropropanol, the use of potassium carbonate was preferable.

### Compound (XII) to Compound (XIII):

The formation of benzoates of formula (XIII) can be accomplished by hydrolysis of nitriles of formula XII using strong acids or bases, an alkali hydroxide, such as e.g. lithiumhydroxide or sodium hydroxide was preferable. As a solvent a polar protic solvent and mixture thereof was suitable such as e.g. an alcohol/water mixture, e.g. ethanol/water, ethanol/water, preferably ethanol/water. The mixure was heated until the reaction was completed. Extraction with a polar aprotic solvent followed by acidification of the remaining aquous phase was performed and the acid (XIII) obtained as a solid.

### Compound (XIII) to Compound (XIV) to Compound (XV):

The compound of formula (XV) can be prepared by reaction of the benzoic acid (XIII) with amines of formula (X) either by
- *in situ* formation of the the corresponding acid chlorides of formula (XIV) and subsequent reaction with amines, preferably with 2-chloro-6-fluoroaniline (VII),
   or by
- amide coupling of the benzoic acid (XV) with amines, preferably with 2-chloro-6-fluoroaniline (VII).

*In situ* formation of acid chlorides of formula (XIV) from benzoic acid (XIII) can be accomplished, for example by using oxalyl chloride or thionyl chloride, both reagents used in the presence of catalytic amount of *N*,*N*-dimethylformamide. In connection with the method according to the invention, oxalyl chloride is preferably used in the presence of *N,N-*dimethylformamide.

Suitable solvents for the *in situ* formation of acid chlorides of formula (XIV) from benzoic acids of formula (XIII) include aprotic nonpolar solvents such as for example dichloromethane or toluene. In connection with the method according to the invention, dichloromethane is preferably used as sovent.

Suitable reaction temperatures for the *in situ* formation of acid chloride (XIV) from benzoic acid (XIII) mostly reflect the boiling point of the solvents used in the reaction. In connection with the method according to the invention, adding of oxalyl chloride was carried out at 0°C and the reaction mixture was subsequently allowed to warm up to room temperature.

Subsequent reactions of the *in situ* formed acid chloride (XIV) with amines, of formula (X) can be carried out in the presence of an organic base. Suitable organic bases are for example triethylamine, pyridine or *N*-ethyl-*N*,*N*-diisopropylamine. In connection with the method according to the invention, triethylamine was preferably used as organic base.

Suitable solvents for the reaction of acid chlorides of formula (XIV) with amines preferably with 2-chloro-6-fluoroaniline (VII), include aprotic polar solvents such as for example acetonitrile, *N*,*N*-dimethylformamide or aprotic nonpolar solvents such as dichloromethane. In conection with the method according to the invention dichoromethane was used as solvent.

Suitable coupling reagents for the reaction of benzoic acid (XIII) with amines are for example *O*-(7-aza-1*H*-benzotriazol-1-yl)-*N*,*N*,*N'*,*N'*-tetramethyluronium hexafluorophosphate (HATU), dicyclohexylcarbodiimide or a combination of 1*H*-benzotria-1-ol and 1-ethyl-3-[3-(diemthylaminopropyl]carbodiimide hydrochloride.

Suitable organic bases for the amide coupling of benzoic acid (XIII) with amines are for example 4-(dimethylamino)pyridine, *N*-ethyl-*N*,*N*-diisopropylamine or triethylamine.

Suitable solvents for the for the amide coupling of benzoic acid (XIII) with amines are for example *N*,*N*-dimethylformamide, dichoromethane or tetrahydrofuran.

For coupling of the amide bond, other methods which are well known to the person skilled in the art are also suitable, such as a condensation between amine and acid using propanephosphonic acid anhydride (T3P) as coupling reagent.

In connection with the invention, formation of compounds (I) and (II), compound of formula (XV) were preferably accomplished by *in situ* formation of acid chlorides of formula (XIV) from benzoic acid (XIII) and subsequent reaction with amines.

### Compound (XV) to Compound (XVII):

Compounds according to the invention of formula (I) can be prepared from halides (XV), preferably wherein A is bromine, and from the triazolone (XVI) using transition metals as catalysts.

Intermediate (XV) can be reacted with the triazolone (XVI), 4-ethyl-5-(hydroxymethyl)-2,4-dihydro-3H-1,2,4-triazol-3-one, in the presence of a suitable base, such as, for example cesium carbonate, and a suitable palladium catalyst, such as for example (1E,4E)-1,5-diphenylpenta-1,4-dien-3-one-palladium, in the presence of a suitable ligand, such as for example (9,9-dimethyl-9*H*-xanthene-4,5-diyl)bis(diphenylphosphine), in a suitable solvent system, such as, for example, dioxane, in a temperature range from room temperature to the boiling point of the respective solvent, preferably the reaction is carried out at 100°C to furnish the compound of formula (II). Alternatively the following palladium catalysts can be used: allylpalladium chloride dimer, dichlorobis(benzonitrile)palladium (II), palladium (II) acetate, palladium (II) chloride, tetrakis(triphenylphosphine)palladium (0), tris(dibenzylideneacetone)dipalladium (0), chloro(2'-amino-1,1'-biphenyl-2-yl)palladium(II) dimer, (2'-amino-1,1'-biphenyl-2-yl)methanesulfonatopalladium(II) dimer, trans-di(µ-acetato)bis[o-(di-o-tolylphosphino)benzyl]dipalladium(II) [cataCXium® C], allylchloro[1,3-bis(2,4,6-trimethylphenyl)imidazol-2-ylidene]palladium(II), allylchloro[1,3-bis(2,6-diisopropylphenyl)imidazol-2-ylidene]palladium(II), chloro[(1,3-dimesitylimidazol-[1,3-bis(2,4,6-trimethylphenyl)-1,3-dihydro-2H-imidazol-2-ylidene](chloro){2-[(dimethylamino)methyl]phenyl} palladium, chloro[(1,2,3-N)-3-phenyl-2-propenyl][1,3-bis(2,6-di-iso-propylphenyl)imidazol-2-ylidene]palladium(II), [2-(acetylamino)phenyl]{1,3-bis[2,6-di(propan-2-yl)phenyl]-1,3-dihydro-2H-imidazol-2-ylidene}chloropalladium, {1,3-bis[2,6-di(propan-2-yl)phenyl]-1,3-dihydro-2H-imidazol-2-ylidene}(chloro){2-[(dimethylamino)methyl]phenyl} palladium, {1,3-bis[2,6-di(propan-2-yl)phenyl]-2,3-dihydro-1H-imidazol-2-yl}(dichloro)(3-chloropyridine-kappaN)palladium, [1,3-bis(2,6-diisopropylphenyl) imidazol-2-ylidene](3-chloropyridyl)palladium(II) dichloride, [2-(acetylamino)-4-methoxyphenyl]{1,3-bis[2,6-di(propan-2-yl)phenyl]-1,3-dihydro-2H-imidazol-2-ylidene}chloropalladium, {1,3-bis[2,6-di(propan-2-yl)phenyl]-1,3-dihydro-2H-imidazol-2-ylidene}(chloro){2-[(dimethylamino)methyl]-3,5-dimethoxyphenyl}palladium, dichloro[1,3-bis(2,6-di-3-pentylphenyl)imidazol-2-ylidene](3-chloropyridyl) palladium(II), dichloro(di-µ-chloro)bis[1,3-bis(2,6-di-iso-propylphenyl) imidazol-2-ylidene]dipalladium(II), 2-(2'-di-tert-butylphosphine)biphenylpalladium(II) acetate, chloro[dicyclohexyl(2',6'-dimethoxybiphenyl-2-yl)-lambda5-phosphanyl][2-(phenyl-kappaC2)ethanaminato-kappaN]palladium, [2-(2-aminoethyl)phenyl](chloro)palladium - di-tert-butyl[2',4',6'-tri(propan-2-yl)biphenyl-2-yl]phosphane, {dicyclohexyl[2',4',6'-tri(propan-2-yl)biphenyl-2-yl]phosphane}{2-[2-(methylazanidyl-kappaN)ethyl]phenyl-kappaC1}palladium, chloro(2-dicyclohexylphosphino-2',6'-dimethoxy-1,1'-biphenyl)(2'-amino-1,1'-biphenyl-2-yl) palladium(II), [2',6'-bis(propan-2-yloxy)biphenyl-2-yl](dicyclohexyl)phosphane - [2-(2-aminoethyl)phenyl](chloro)palladium, [2-(2-aminoethyl)phenyl](chloro){dicyclohexyl[2',4',6'-tri(propan-2-yl)biphenyl-2-yl]-lambda5-phosphanylidene}palladium, 2'-(dicyclohexylphosphanyl)-N,N,N',N'-tetramethylbiphenyl-2,6-diamine - (2'-aminobiphenyl-2-yl)(chloro)palladium, chloro(2-dicyclohexylphosphino-2',6'-di-iso-propoxy-1,1'-biphenyl)(2-amino-1,1'-biphenyl-2-yl)palladium(II), [2'-(azanidyl-kappaN)biphenyl-2-yl-kappaC2](chloro){dicyclohexyl [2',4',6'-tri(propan-2-yl)biphenyl-2-yl]-lambda5-phosphanyl}palladium, (2'-aminobi-phenyl-2-yl)(methanesulfonato-kappaO)palladium - di-tert-butyl[2',4',6'-tri(propan-2-yl)biphenyl-2-yl]phosphane, (2'-aminobiphenyl-2-yl)palladium(1+) methanesulfonate-di-tert-butyl[2',4',6'-tri(propan-2-yl)biphenyl-2-yl]phosphane, dicyclohexyl[3,6-dimethoxy-2',4',6'-tri(propan-2-yl)biphenyl-2-yl]phosphane-[2-(2-aminoethyl) phenyl](chloro)palladium, (2'-aminobiphenyl-2-yl)palladium(1+) methanesulfonate-2'-(dicyclohexylphosphanyl)-N,N,N',N'-tetramethylbiphenyl-2,6-diamine, sodium 2'-(dicyclohexylphosphanyl)-2,6-dimethoxybiphenyl-3-sulfonate-(2'-aminobiphenyl-2-yl)(chloro)palladium, chloro(2-dicyclohexylphosphino-2',4',6'-tri-iso-propyl-1,1'-biphenyl)[2-(2-aminoethyl)phenyl]palladium(II), (2'-aminobiphenyl-2-yl)(methane-sulfonato-kappaO)palladium - [2',6'-bis(propan-2-yloxy)biphenyl-2-yl](dicyclohexyl)phosphane, (2'-aminobiphenyl-2-yl)(methanesulfonato-kappaO)palladium - dicyclohexyl[2',4',6'-tri(propan-2-yl)biphenyl-2-yl]phosphane, (2'-aminobiphenyl-2-yl)palladium(1+) methanesulfonate - dicyclohexyl[2',4',6'-tri(propan-2-yl)biphenyl-2-yl]phosphane, dicyclohexyl[3,6-dimethoxy-2',4',6'-tri(propan-2-yl)biphenyl-2-yl]phosphane - (2'-aminobiphenyl-2-yl)(chloro)palladium, (2'-aminobiphenyl-2-yl)(methanesulfonato-kappaO)palladium - di-tert-butyl[3,6-dimethoxy-2',4',6'-tri(propan-2-yl)biphenyl-2-yl]phosphane, (2'-aminobiphenyl-2-yl)(methanesulfonato-kappaO)palladium - dicyclohexyl[3,6-dimethoxy-2',4',6'-tri(propan-2-yl)biphenyl-2-yl]phosphane or the following ligands:
racemic-2,2'-bis(diphenylphosphino)-1,1'-binaphthyl, rac-BINAP, 1,1'-bis(diphenyl-phosphino)ferrocene, bis(2-diphenylphosphinophenyl)ether, di-*tert*-butylmethylphosphonium tetrafluoroborate, 2-(di-*tert*-butylphosphino)biphenyl, tri-*tert*-butylphosphonium tetrafluoroborate, tri-2-furylphosphine, tris(2,4-di-*tert*-butylphenyl)phosphite, tri-o-tolylphosphine, (9,9-dimethyl-9*H*-xanthene-4,5-diyl)bis(diphenylphosphine), dicyclohexyl(2',4',6'-triisopropyl-3,6-dimethoxybiphenyl-2-yl)phosphine, di-tert-butyl (2',4',6'-triisopropyl-3,6-dimethoxybiphenyl-2-yl)phosphine, di-tert-butyl(2',4',6'-triiso propylbiphenyl-2-yl)phosphine, dicyclohexyl(2',4',6'-triisopropylbiphenyl-2-yl) phosphine, di-tert-butyl(2',4',6'-triisopropyl-3-methoxy-6-methylbiphenyl-2-yl)phos-phine, di-tert-butyl(2',4',6'-triisopropyl-3,4,5,6-tetramethylbiphenyl-2-yl) phosphine, adamantan-1-yl(adamantan-2-yl)(2',4',6'-triisopropyl-3,6-dimethoxybiphenyl-2-yl) phosphine, dicyclohexyl(2',6'-dimethoxybiphenyl-2-yl)phosphine, dicyclohexyl(2',6'-diisopropoxybiphenyl-2-yl)phosphine, 2'-(dicyclohexylphosphino)-N,N-dimethyl-biphenyl-2-amine, 2'-(di-tert-butylphosphino)-N,N-dimethylbiphenyl-2-amine, 2'-(di-phenylphosphino)-N,N,N',N'-tetramethylbiphenyl-2,6-diamine, di-tert-butyl(2',4',6'-tricyclohexyl-3,6-dimethoxybiphenyl-2-yl)phosphine, bis[3,5-bis(trifluoromethyl)phe-nyl] (2',4',6'-triisopropyl-3,6-dimethoxybiphenyl-2-yl)phosphine, biphenyl-2-yl(di-tert-butyl)phosphine, dicyclohexyl(2'-methylbiphenyl-2-yl)phosphine, biphenyl-2-yl (dicyclohexyl)phosphine, 2'-(dicyclohexylphosphino)-N,N-dimethylbiphenyl-2-amine, 2'-(dicyclohexylphosphino)-N,N,N',N'-tetramethylbiphenyl-2,6-diamine, sodium 2'-(dicyclohexylphosphino)-2,6-diisopropylbiphenyl-4-sulfonate, sodium 2'-(dicyclohexylphosphino)-2,6-dimethoxybiphenyl-3-sulfonate, 1,1'-binaphthalen-2-yl(di-tert-butyl)phosphine, 1,3-bis(2,4,6-trimethylphenyl)-1,3-dihydro-2H-imidazol-2-ylidene, 1,3-bis[2,6-di(propan-2-yl)phenyl]-1,3-dihydro-2H-imidazol-2-ylidene.

### (Compound (XVII) to Compound (II):

Microbiologic transformation of Compound (XVII) can be performed by using readily available hepatocytes from different species which after incubation with William's E medium were contacted with compound (XVII). HPLC of the resulting mixture after stopping the reaction by addition of an organic solvent results in obtaining Compound (II).

In accordance with a second aspect, the present invention provides methods of preparing compound (I) as defined *supra,* said methods comprising the step of allowing an intermediate compound of formula (VIII) : 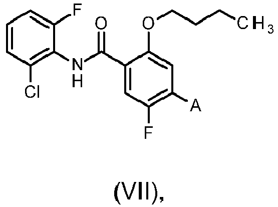 in which A is chlorine, bromine or iodine,
to react with a compound of formula (IX) : 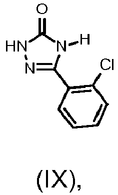 thereby giving a compound of formula (I) :

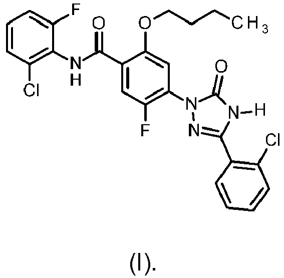

In accordance with a third aspect, the present invention provides a method of preparing compound (II) as defined *supra,* said methods comprising the step of allowing an intermediate compound of formula (XVII) : 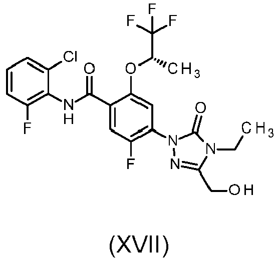 to be converted into 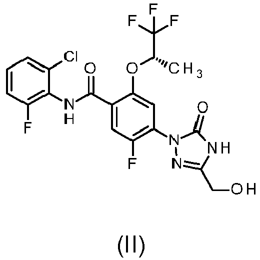 by a biological method.

In one embodiment said biological method comprises contacting compound (XVII) with hepatocytes, stopping the reaction by addition of an organic solvent and isolating the product by chromatography.

The present invention provides methods of preparing compounds of the present invention of formula (I) and (II), said methods comprising the steps as described in the Experimental Section herein.

In accordance with a fourth aspect, the present invention provides intermediate compounds which are useful for the preparation of the compounds (I) and (II), *supra.*

Particularly, the invention provides the intermediate compound (VII) : 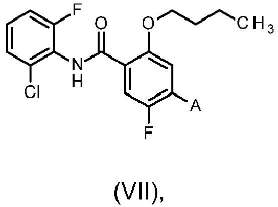 in which A is a chlorine, bromine or iodine atom, preferably a bromine atom.

In accordance with a fifth aspect, the present invention provides the use of said intermediate compounds for the preparation of a compound of formula (I) and (II) as defined *supra.*

According to another aspect, the invention provides the intermediate compound (VII) : 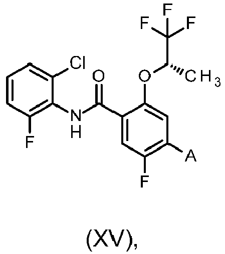 in which A is a chlorine, bromine or iodine atom, preferably a bromine atom.

The present invention provides the intermediate compounds which are disclosed in the Example Section of this text, *infra.*

The compounds (I) or (II) of the present invention can be converted to any salt, preferably pharmaceutically acceptable salts, as described herein, by any method which is known to the person skilled in the art. Similarly, any salt of a compound of formula (I) or (II) of the present invention can be converted into the free compound, by any method which is known to the person skilled in the art.

Compounds (I) or (II) of the present invention demonstrate a valuable pharmacological action. Compounds of the present invention have surprisingly been found to effectively inhibit dihydroorotate dehydrogenase (DHODH) and it is possible therefore that said compounds be used for the treatment or prophylaxis of diseases, preferably hyperproliferative disorders.

Compounds (I) and (II) of the present invention can be utilized to inhibit, block, reduce, decrease, cell proliferation and/or cell division. This method comprises administering to a mammal in need thereof, including a human, an amount of compound (I) or (II) of the present invention, or a pharmaceutically acceptable salt, isomer, polymorph, metabolite, hydrate, solvate or ester thereof, which is effective to treat the disorder.

Hyperproliferative disorders include, but are not limited to, for example : psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancers include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, medulloblastoma, ependymoma, as well as neuroectodermal and pineal tumour.

Tumours of the male reproductive organs include, but are not limited to, prostate and testicular cancer.

Tumours of the female reproductive organs include, but are not limited to, endometrial, cervical, ovarian, vaginal, and vulvar cancer, as well as sarcoma of the uterus.

Tumours of the digestive tract include, but are not limited to, anal, colon, colorectal, oesophageal, gallbladder, gastric, pancreatic, rectal, small-intestine, and salivary gland cancers.

Tumours of the urinary tract include, but are not limited to, bladder, penile, kidney, renal pelvis, ureter, urethral and human papillary renal cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal, hypopharyngeal, nasopharyngeal, oropharyngeal cancer, lip and oral cavity cancer and squamous cell.

Lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

The present invention also provides methods of treating angiogenic disorders including diseases associated with excessive and/or abnormal angiogenesis.

Inappropriate and ectopic expression of angiogenesis can be deleterious to an organism. A number of pathological conditions are associated with the growth of extraneous blood vessels. These include, for example, diabetic retinopathy, ischemic retinal-vein occlusion, and retinopathy of prematurity [Aiello et al., New Engl. J. Med., 1994, 331, 1480 ; Peer et al., Lab. Invest., 1995, 72, 638], age-related macular degeneration (AMD) [Lopez et al., Invest. Opththalmol. Vis. Sci., 1996, 37, 855], neovascular glaucoma, psoriasis, retrolental fibroplasias, angiofibroma, inflammation, rheumatoid arthritis (RA), restenosis, in-stent restenosis, vascular graft restenosis, *etc.* In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of formula (I) or (II) of the present invention can be utilized to treat and/or prevent any of the aforementioned angiogenesis disorders, for example by inhibiting and/or reducing blood vessel formation; by inhibiting, blocking, reducing, decreasing, *etc.* endothelial cell proliferation, or other types involved in angiogenesis, as well as causing cell death or apoptosis of such cell types.

These disorders have been well characterized in humans, but also exist with a similar etiology in other mammals, and can be treated by administering pharmaceutical compositions of the present invention.

The term "treating" or "treatment" as stated throughout this document is used conventionally, for example the management or care of a subject for the purpose of combating, alleviating, reducing, relieving, improving the condition of a disease or disorder, such as a carcinoma.

The compounds of the present invention can be used in particular in therapy and prevention, i.e. prophylaxis, of tumour growth and metastases, especially in solid tumours of all indications and stages with or without pre-treatment of the tumour growth.

Generally, the use of chemotherapeutic agents and/or anti-cancer agents in combination with a compound or pharmaceutical composition of the present invention will serve to:
1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of formula (I) or (II) of the present invention can also be used in combination with radiotherapy and/or surgical intervention.

In a further embodiment of the present invention, the compounds of formula (I) or (II) of the present invention may be used to sensitize a cell to radiation, i.e. treatment of a cell with a compound of the present invention prior to radiation treatment of the cell renders the cell more susceptible to DNA damage and cell death than the cell would be in the absence of any treatment with a compound of the present invention. In one aspect, the cell is treated with one compound of formula (I) or (II) of the present invention.

Thus, the present invention also provides a method of killing a cell, wherein a cell is administered one or more compounds of the present invention in combination with conventional radiation therapy.

The present invention also provides a method of rendering a cell more susceptible to cell death, wherein the cell is treated with one or more compounds of formula (I) or (II) of the present invention prior to the treatment of the cell to cause or induce cell death. In one aspect, after the cell is treated with one or more compounds of formula (I) or (II) of the present invention, the cell is treated with at least one compound, or at least one method, or a combination thereof, in order to cause DNA damage for the purpose of inhibiting the function of the normal cell or killing the cell.

In other embodiments of the present invention, a cell is killed by treating the cell with at least one DNA damaging agent, i.e. after treating a cell with one or more compounds of formula (I) or (II) of the present invention to sensitize the cell to cell death, the cell is treated with at least one DNA damaging agent to kill the cell. DNA damaging agents useful in the present invention include, but are not limited to, chemotherapeutic agents (e.g. cis platin), ionizing radiation (X-rays, ultraviolet radiation), carcinogenic agents, and mutagenic agents.

In other embodiments, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In one aspect of the invention, a compound of formula (I) or (II) of the present invention is administered to a cell prior to the radiation or other induction of DNA damage in the cell. In another aspect of the invention, a compound of formula (I) or (II) of the present invention is administered to a cell concomitantly with the radiation or other induction of DNA damage in the cell. In yet another aspect of the invention, a compound of formula (I) or (II) of the present invention is administered to a cell immediately after radiation or other induction of DNA damage in the cell has begun.

In another aspect, the cell is in vitro. In another embodiment, the cell is in vivo.

Compounds of the present invention can be utilized to inhibit, block, reduce, dihydroorotate dehydrogenase. This method comprises administering to a mammal in need thereof, including a human, an amount of compound (I) or (II), or a pharmaceutically acceptable salt, isomer, polymorph, metabolite, hydrate, solvate or ester thereof; which is effective to treat the disorder.

The present invention also provides methods of treating hyperproliferative disorders, especially cancer.

These disorders have been well characterized in humans, but also exist with a similar etiology in other mammals, and can be treated by administering pharmaceutical compositions of the present invention.

The term "treating" or "treatment" as used in the present text is used conventionally, e.g., the management or care of a subject for the purpose of combating, alleviating, reducing, relieving, improving the condition of a disease or disorder, such as a carcinoma.

The compounds of the present invention can be used in particular in therapy and prevention, i.e. prophylaxis, of hyperproliferative disorders, especially cancer.

In accordance with a further aspect, the present invention provides compounds (I) and (II), as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular hyperproliferative disorders, especially cancer, more particularly the cancer disorders as mentioned herein.

The pharmaceutical activity of the compounds according to the invention can be explained by their activity as inhibitors of dihydroorotate dehydrogenase.

In accordance with a further aspect, the present invention provides the use of compounds (I) and (II), as described *supra,* or stereoisomers, tautomers, N-oxides, hydrates, solvates, and salts thereof, particularly pharmaceutically acceptable salts thereof, or mixtures of same, for the treatment or prophylaxis of diseases, in particular hyperproliferative disorders, particularly cancer disorders.

In accordance with a further aspect, the present invention provides the use of compounds (I) and (II), described *supra,* or a stereoisomer, a tautomer, an N-oxide, a hydrate, a solvate, or a salt thereof, particularly a pharmaceutically acceptable salt thereof, or a mixture of same, for the prophylaxis or treatment of diseases, in particular hyperproliferative disorders, particularly cancer disorders.

In accordance with a further aspect, the present invention provides the use of compounds (I) and (II), as described *supra,* or stereoisomers, tautomers, N-oxides, hydrates, solvates, and salts thereof, particularly pharmaceutically acceptable salts thereof, or mixtures of same, in a method of treatment or prophylaxis of diseases, in particular hyperproliferative disorders, particularly cancer disorders.

In accordance with a further aspect, the present invention provides use of compounds (I) and (II), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer disorders.

In accordance with a further aspect, the present invention provides a method of treatment or prophylaxis of diseases, in particular hyperproliferative disorders, particularly cancer disorders, using an effective amount of compounds (I) and (II), as described *supra,* or stereoisomers, tautomers, N-oxides, hydrates, solvates, and salts thereof, particularly pharmaceutically acceptable salts thereof, or mixtures of same.

In accordance with a further aspect, the present invention provides pharmaceutical compositions, in particular a medicament, comprising compounds (I) and (II), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore provides pharmaceutical compositions, in particular medicaments, which comprise at least compound (I) or (II), conventionally together with one or more pharmaceutically suitable excipients, and to their use for the above mentioned purposes.

It is possible for compounds (I) and (II) to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for compounds (I) and (II) to be administered in suitable administration forms.

For oral administration, it is possible to formulate compounds (I) and (II) to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

Compounds (I) and (II) can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),
- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least compounds (I) or (II), conventionally together with one or more pharmaceutically suitable excipient(s), and to their use according to the present invention.

In accordance with another aspect, the present invention provides pharmaceutical combinations, in particular medicaments, comprising compound (I) or (II) and at least one or more further active ingredients, in particular for the treatment and/or prophylaxis of a hyperproliferative disorder, cancer disorder.

Particularly, the present invention provides a pharmaceutical combination, which comprises:
- one or more first active ingredients, in particular compound (I) or (II) as defined *supra,* and
- one or more further active ingredients, in particular an anti-cancer agent.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of formula (I) or (II) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

The compounds of the present invention can be administered as the sole pharmaceutical agent or in combination with one or more other pharmaceutically active ingredients where the combination causes no unacceptable adverse effects. The present invention also provides such pharmaceutical combinations. For example, the compounds of the present invention can be combined with known anti-cancer agents.

Examples of indication agents include: 131I-chTNT, abarelix, abemaciclib, abiraterone, acalabrutinib, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, apalutamide, aprepitant, arcitumomab, arglabin, arsenic trioxide, asparaginase, atezolizumab, avelumab, axicabtagene ciloleucel, axitinib, azacitidine, basiliximab, belotecan, bendamustine, besilesomab, belinostat, bevacizumab, bexarotene, bicalutamide, bisantrene, bleomycin, blinatumomab, bortezomib, bosutinib, buserelin, brentuximab vedotin, brigatinib, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, durvalumab, eculizumab, edrecolomab, elliptinium acetate, elotuzumab, eltrombopag, enasidenib, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, inotuzumab ozogamicin, interferon alfa, interferon beta, interferon gamma, iobitridol, iobenguane (1231), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, lutetium Lu 177 dotatate, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, midostaurin, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, mvasi, nabilone, nabiximols, nafarelin, naloxone + pentazocine, naltrexone, nartograstim, necitumumab, nedaplatin, nelarabine, neratinib, neridronic acid, netupitant/palonosetron, nivolumab, pentetreotide, nilotinib, nilutamide, nimorazole, nimotuzumab, nimustine, nintedanib, niraparib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, ribociclib, risedronic acid, rhenium-186 etidronate, rituximab, rolapitant, romidepsin, romiplostim, romurtide, rucaparib, samarium (153Sm) lexidronam, sargramostim, sarilumab, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

Based upon standard laboratory techniques known to evaluate compounds useful for the treatment of hyperproliferative disorders, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### EXPERIMENTAL SECTION

NMR peak forms are stated as they appear in the spectra, possible higher order effects have not been considered.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| The following table lists the abbreviations used herein. | |
|---|---|
| a.u. | Arbitrary units |
| µM | micromolar |
| µL | microliter |
| µm | micrometer |
| Da | dalton |
| DAD | diode array detector |
| DCM | dichloromethane |
| DMAP | 4-(dimethylamino)pyyidine |
| ESI | electrospray ionization |
| FCS | Fetal Calf Serum |
| h | hour(s) |
| HPLC | high performance liquid chromatography |
| HPLC-MS | high performance liquid chromatography/mass spectrometry |
| HRMS | high resolution mass spectrometry |
| KHMDS | potassium-bis(trimethylsilyl)amide |
| LC | liquid chromatography |
| LC-MS | liquid chromatography-mass spectrometry |
| m/z | mass to charge ratio |
| min | minute(s) |
| mM | millimolar |
| MS | mass spectrometry |
| MS/MS | tandem mass spectrometry |
| No. | number |
| Rt | retention time |
| THF | tetrahydrofurane |
| tBuBrettphos | 2-(Di-tert-butylphosphino)-2',4',6'-triisopropyl-3,6-dimethoxy-1,1'-biphenyl |
| tBuBrettphos-G3-Pd | [(2-Di-tert-butylphosphino-3,6-dimethoxy-2',4',6'-triisopropyl-1,1'-biphenyl)-2-(2'-amino-1,1'-biphenyl)]palladium(II) methanesulfonate |
| UV | ultraviolett |

The various aspects of the invention described in this application are illustrated by the following examples which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### UPLC-MS Standard Procedures

Analytical UPLC-MS was performed as described below. The masses (m/z) are reported from the positive mode electrospray ionisation unless the negative mode is indicated (ESI-). In most of the cases method 1 is used. If not, it is indicated.

### Method 1:

Instrument: Waters Acquity UPLC-MS SQD 3001; Column: Acquity UPLC BEH C18 1.7 50x2.1mm; Eluent A: water + 0.2% ammonia, Eluent B: acetonitrile; Gradient: 0-1.6 min 1-99% B, 1.6-2.0 min 99% B; Flow rate 0.8 mL/min; Temperature: 60°C; Injection: 2 µL; DAD scan: 210-400 nm; ELSD.

### Method 2:

Instrument: Waters Acquity UPLC-MS SQD 3001; Column: Acquity UPLC BEH C18 1.7 50x2.1mm; Eluent A: water + 0.1% formic acid , Eluent B: acetonitrile; Gradient: 0-1.6 min 1-99% B, 1.6-2.0 min 99% B; Flow rate 0.8 mL/min; Temperature: 60L:°C; Injection: 2 µL; DAD scan: 210-400 nm.

### Method 3:

Instrument: Waters Acquity UPLCMS SingleQuad; Column: Acquity UPLC BEH C18 1.7 µm, 50x2.1mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm.

### EXPERIMENTAL SECTION

### EXPERIMENTAL SECTION - INTERMEDIATES

### Intermediate 1

### tert-butyl 4-bromo-2,5-difluorobenzoate

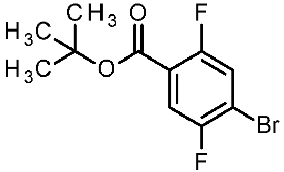

To stirred solution of 4-bromo-2,5-difluorobenzoic acid (10.0 g, 42.2 mmol) in tert-butanol (200 mL) was successively added di-tert-butyl dicarbonate (18.4 g, 84.4 mmol) and DMAP (515 mg, 4.22 mmol). The resulting mixture was stirred 35 °C overnight and concentrated under reduced pressure. The residue was diluted with ethyl acetate and washed with aqueous 1.0 M hydrochloric acid, saturated aqueous sodium bicarbonate, brine, dried (sodium sulfate) and concentrated under reduced pressure. The residue was sufficiently clean to use in the next step (11.6 g, 94%).
¹H-NMR (400 MHz, DMSO-d6) δ [ppm]: 1.370 (0.40), 1.376 (0.47), 1.526 (16.00), 7.730 (0.44), 7.745 (0.48), 7.751 (0.48), 7.766 (0.44), 7.868 (0.44), 7.882 (0.45), 7.892 (0.45), 7.906 (0.44).

### Intermediate 2

### 4-bromo-2-butoxy-5-fluorobenzoic acid

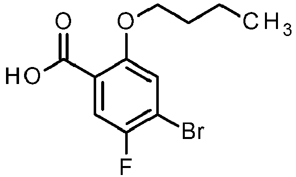

Butan-1-ol (1.2 ml, 14 mmol) was dissolved in THF (12 mL) and cooled to -10 °C. KHMDS solution (7.4 ml, 1.0 M, 7.4 mmol) was added dropwise and the mixture was stirred at -10 °C for 1h. This solution was added dropwise to a solution of tert-butyl 4-bromo-2,5-difluorobenzoate (Intermediate 1, 1.80 g, 6.14 mmol) in THF (6 mL) cooled to -10 °C. The mixture was stirred for 1h at -10 °C. The reaction was quenched with water and DCM. The phases were separated and the aqueous phase was extracted with DCM. The combined organic phases were washed with brine, dried over sodium sulfate and concentrated to yield butyl 4-bromo-2-butoxy-5-fluorobenzoate as a yellow oil (1.73g).

The yellow oil was dissolved in dioxane (9 mL). Water (2 mL) and lithium hydroxide (362 mg, 15.1 mmol) was added. The resulting mixture was stirred at room temperature overnight. The mixture was diluted with water and acidified to pH 2 with 2N aqueous hydrochloric acid. The aqueous phase was extracted with DCM twice. The combined organic extracts were dried (sodium sulfate) and concentrated under reduced pressure to give the desired product (1,36 g, 93%).
LC-MS (Method 3): Rₜ = 1.26 min; MS (ESIneg): m/z = 289 [M-H]⁻
¹H-NMR (400 MHz, DMSO-d6) δ [ppm]: 0.894 (7.07), 0.912 (16.00), 0.930 (8.05), 1.389 (0.58), 1.407 (2.18), 1.425 (3.64), 1.444 (3.75), 1.463 (2.29), 1.481 (0.61), 1.632 (1.15), 1.648 (2.99), 1.667 (3.54), 1.684 (2.74), 1.700 (0.85), 3.361 (0.57), 3.377 (0.62), 3.565 (1.47), 4.016 (3.98), 4.032 (8.21), 4.048 (3.91), 7.448 (4.71), 7.462 (4.69), 7.553 (5.60), 7.575 (5.59), 13.015 (0.58).

### Intermediate 3

### 4-bromo-2-butoxy-N-(2-chloro-6-fluorophenyl)-5-fluorobenzamide

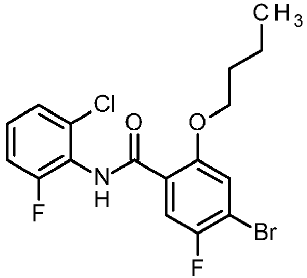

To a stirred solution of 4-bromo-2-butoxy-5-fluorobenzoic acid (Intermediate 2, 1.36 g, 4.67 mmol) and catalytic DMF in anhydrous DCM (25 mL) was added oxalyl chloride (490 µl, 5.6 mmol) dropwise. The resulting mixture was stirred for 1 h and concentrated under reduced pressure. A solution of the residue in anhydrous DCM (20 mL) was added dropwise to a stirred solution of 2-chloro-6-fluoroaniline (750 mg, 5.15 mmol) and triethylamine (720 µl, 5.2 mmol) in anhydrous DCM (30 mL). Following complete addition, the mixture stirred for 1 h. The mixture was concentrated under reduced pressure. The residue was dissolved in ethanol (15 mL) and precipitated by slow addition of water (30 mL). The solid was collected on a filter, washed with water and dried in a vacuum oven to yield 1.73 g (88 % yield) of the desired product.
LC-MS: Rₜ = 1.55 min; MS (ESIpos): m/z = 418 [M+H]⁺
¹H-NMR (400 MHz, DMSO-d6) δ [ppm]: 0.787 (0.48), 0.889 (6.64), 0.908 (16.00), 0.926 (7.83), 1.390 (0.52), 1.408 (1.83), 1.427 (3.10), 1.446 (3.07), 1.464 (1.83), 1.483 (0.50), 1.731 (0.76), 1.747 (2.12), 1.767 (2.57), 1.784 (2.05), 1.801 (0.67), 2.518 (3.81), 2.523 (2.64), 4.165 (2.33), 4.181 (4.62), 4.197 (2.31), 7.329 (0.79), 7.348 (1.90), 7.367 (1.26), 7.372 (1.43), 7.380 (1.00), 7.395 (0.95), 7.400 (1.79), 7.414 (1.86), 7.421 (1.38), 7.433 (4.14), 7.438 (3.57), 7.453 (1.07), 7.458 (0.93), 7.579 (2.69), 7.593 (2.74), 7.612 (3.05), 7.634 (2.88), 9.836 (4.40).

### Intermediate 4

### 4-bromo-5-fluoro-2-{[(2S)-1,1,1-trifluoropropan-2-yl]oxy}benzonitrile

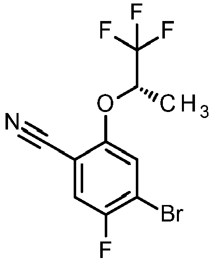

To a stirred suspension of 4-bromo-2,5-difluorobenzonitrile (91 g, 417 mmol) and potassium carbonate (173 g, 1.25 mol) in N,N-dimethylformamide (910 ml) was added (S)-1,1,1-trifluoropropanol [CAS 3539-97-7] dropwise (52.4 g, 460 mmol). The resulting mixture was heated at 70 °C for for 15 hours and cooled to room temperature. The reaction was concentrated and the residue was diluted with water. The aqueous solution was extracted with DCM (3x). The combined organic washes were washed with brine, dried over sodium sulfate, filtered, and concentrated under reduced pressure to give an oil (136.6 g). The residue was triturated with a mixture of hexanes and toluene (9:1, 200 mL) to give the desired product as a white solid (90.3 g, 93% purity, 64% yield).
LC-MS (Method A): Rₜ = 1.29 min; MS (ESIpos): m/z = 312.0 [M+H]⁺.
¹H-NMR (400 MHz, CHLOROFORM-d) δ [ppm]: 1.511 (4.41), 1.531 (15.64), 1.533 (16.00), 1.548 (15.84), 1.550 (15.83), 4.566 (1.08), 4.581 (2.68), 4.597 (3.18), 4.612 (2.62), 4.626 (1.01), 7.194 (5.27), 7.207 (7.60), 7.220 (7.57).

### Intermediate 5

### 4-bromo-5-fluoro-2-{[(2S)-1,1,1-trifluoropropan-2-yl]oxy}benzoic acid

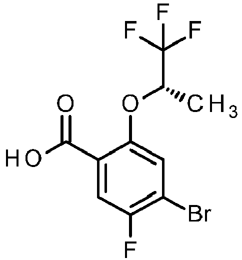

To a solution of 4-bromo-5-fluoro-2-{[(2S)-1,1,1-trifluoropropan-2-yl]oxy}benzonitrile (Intermediate 4, 27.4 g, 87.9 mmol) in ethanol (90 ml) was added aqueous sodium hydroxide (2 N, 140 ml) and the resulting mixture was heated to 90 °C for 20 hours. The resulting solution was cooled to room temperature, diluted with water, and extracted with dichloromethane. The aqueous phase was acidified with 2 N aqueous hydrochloric acid (pH 2) upon which a white solid precipitated. The suspension was stirred for further 15 minutes, the solid was filtered off, washed with water and dried *in vacuo.* to yield an off-white solid (25.97 g, 89 %), which was used for the next step without further purification.
LC-MS (Method A): Rₜ = 1.16 min; MS (ESIpos): m/z = 331 [M+H]⁺.
¹H-NMR (400 MHz, DMSO-d6) δ [ppm]: 1.396 (15.96), 1.411 (16.00), 1.469 (0.69), 1.484 (0.45), 2.518 (3.48), 2.523 (2.36), 5.288 (1.19), 5.304 (2.89), 5.320 (3.73), 5.336 (2.70), 5.352 (1.05), 7.612 (11.35), 7.634 (11.12), 7.743 (7.88), 7.757 (7.91).

### Intermediate 6

### 4-bromo-5-fluoro-2-{[(2S)-1,1,1-trifluoropropan-2-yl]oxy}benzoyl chloride

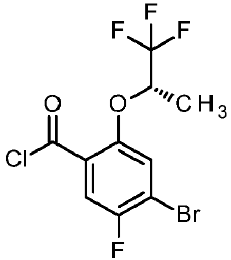

To a solution of 4-bromo-5-fluoro-2-{[(2S)-1,1,1-trifluoropropan-2-yl]oxy}benzoic acid (Intermediate 5, 15.0 g, 45.3 mmol) in dichloromethane (230 ml) was added N,N-dimethylformamide (350 µl), followed by dropwise addition of ethanedioyl dichloride (4.7 ml, 54 mmol). The reaction mixture was stirred at room temperature for one hour, and concentrated under reduced pressure. The title compound was obtained as brown oil (15.84 g, quantitative), which was used for the next step without purification. For analytic, a small amount of the product was treated with methanol, to yield the corresponding methyl ester, which was detected by LC-MS.
LC-MS (Method A) [methyl ester]: Rₜ = 1.36 min; MS (ESIpos): m/z = 345 [M+H]⁺.

### Intermediate 7

### 4-bromo-N-(2-chloro-6-fluorophenyl)-5-fluoro-2-{[(2S)-1,1,1-trifluoropropan-2-yl]oxy}benzamide

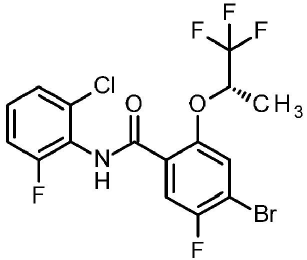

4-Bromo-5-fluoro-2-{[(2S)-1,1,1-trifluoropropan-2-yl]oxy}benzoyl chloride (Intermediate 6, 31.9 g, 91.3 mmol) was dissolved in DCM (300 mL) and added to a solution of 2-chloro-6-fluoroaniline (14.6 g, 100.4 mmol) and triethylamine (14 ml, 100 mmol) in DCM (400 mL).The mixture was stirred at room temperature for 30 min. The mixture was concentrated to yield 47.9g of crude product. The crude product was dissolved in ethanol (250 mL) and water (500 mL) was added slowly. The resulting precipitate was filtered off, the solids were washed with water and dried to yield the desired product (39.3 g, 84.5 % yield).
LC-MS (Method A): Rₜ = 1.44 min; MS (ESIpos): m/z = 458 [M+H]⁺.
¹H-NMR (400 MHz, DMSO-d6) δ [ppm]: 1.343 (1.46), 1.359 (1.66), 1.387 (0.50), 1.437 (16.00), 1.452 (15.88), 1.475 (0.73), 1.495 (0.82), 1.513 (0.53), 1.907 (0.67), 2.332 (1.23), 2.518 (7.59), 2.523 (4.64), 2.673 (1.20), 5.400 (1.28), 5.416 (2.77), 5.432 (3.47), 5.448 (2.51), 5.464 (1.02), 7.323 (1.55), 7.328 (1.69), 7.347 (3.94), 7.354 (2.01), 7.363 (2.31), 7.371 (2.98), 7.383 (2.19), 7.395 (1.78), 7.402 (3.88), 7.416 (5.31), 7.425 (9.08), 7.431 (9.78), 7.445 (2.16), 7.481 (0.67), 7.502 (0.44), 7.528 (6.83), 7.549 (6.80), 7.665 (0.44), 7.680 (0.70), 7.694 (0.41), 7.820 (5.90), 7.833 (5.87), 9.977 (11.65).

### Intermediate 8

### 4-ethyl-5-(hydroxymethyl)-2,4-dihydro-3H-1,2,4-triazol-3-one

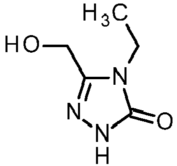

2-hydroxyacetohydrazide (50.0 g, 555 mmol) was dissolved in water (125 mL) and cooled to 0°C. Ethyl isocyanate (44 ml, 560 mmol) was added (very exotherm!) and the resulting mixture was stirred at room temperature overnight. The resulting suspension was treated with sodium hydroxide solution (64g, 50 wt% in water) (exotherm). The resulting solution was heated to 95 °C overnight. The yellow reaction mixture was neutralized with concentrated hydrochloric acid and the resulting cloudy solution was concentrated to dryness. The solids were triturated with a mixture of dichloromethane and isopropanol (4:1, 750 mL), the solution was filtered off and concentrated to dryness again to yield ∼78g of crude product. The product was recrystallized from ethyl acetate to yield the desired product (56.2g, 71% yield).
¹H-NMR (400 MHz, DMSO-d6) δ [ppm]: 1.156 (6.88), 1.174 (16.00), 1.192 (7.02), 2.518 (0.48), 3.606 (2.02), 3.623 (6.84), 3.641 (6.79), 3.659 (1.96), 4.316 (4.51), 5.540 (0.94).

### EXPERIMENTAL SECTION - EXAMPLES

### Example 1

### 2-butoxy-N-(2-chloro-6-fluorophenyl)-4-[3-(2-chlorophenyl)-5-oxo-4,5-dihydro-1H-1,2,4-triazol-1-yl]-5-fluorobenzamide (Compound I)

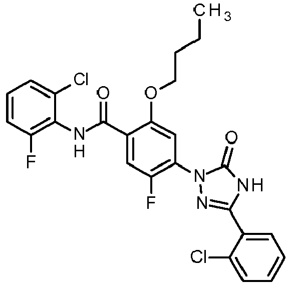

4-bromo-2-butoxy-N-(2-chloro-6-fluorophenyl)-5-fluorobenzamide (Intermediate 3, 150 mg, 358 µmol), 5-(2-chlorophenyl)-2,4-dihydro-3H-1,2,4-triazol-3-one (84.1 mg, 430 µmol), cesium carbonate (233 mg, 717 µmol), and powdered molecular sieves (3Å) were loaded into a microwave vial. The vial was purged with argon, dioxane (4 mL, degassed) was added, followed by tBuBrettPhos (5.21 mg, 10.7 µmol), tBuBrettphos-G3-Pd (9.18 mg, 10.7 µmol). The vial was sealed and the mixture was stirred for 3h at 70°C. An additional amount of tBuBrettPhos (5.21 mg, 10.7 µmol) and tBuBrettphos-G3-Pd (9.18 mg, 10.7 µmol) was added and and the mixture was stirred overnight at 70°C. The resulting suspension was filtered over Celite, washed with THF and concentrated. Reverse phase preparative chromatography yielded the desired product (41.0 mg, 21 % yield).
LC-MS: Rₜ = 1.42 min; MS (ESIpos): m/z = 533 [M+H]⁺
¹H-NMR (400 MHz, DMSO-d6) δ [ppm]: 0.894 (6.83), 0.913 (16.00), 0.931 (7.76), 1.407 (0.52), 1.425 (1.85), 1.444 (3.15), 1.463 (3.14), 1.481 (1.88), 1.500 (0.49), 1.758 (0.78), 1.774 (2.17), 1.794 (2.63), 1.812 (2.04), 1.828 (0.65), 1.907 (0.54), 2.084 (0.93), 2.518 (4.31), 2.523 (2.76), 4.163 (2.32), 4.179 (4.59), 4.195 (2.26), 5.758 (1.68), 7.343 (0.83), 7.363 (2.04), 7.382 (1.45), 7.387 (1.77), 7.405 (1.08), 7.410 (1.94), 7.424 (1.94), 7.431 (1.37), 7.447 (3.50), 7.451 (3.50), 7.467 (1.34), 7.471 (1.31), 7.483 (2.55), 7.498 (2.50), 7.511 (1.39), 7.514 (1.42), 7.529 (3.19), 7.533 (3.19), 7.548 (2.44), 7.551 (2.45), 7.575 (1.73), 7.579 (1.88), 7.595 (2.79), 7.599 (3.07), 7.613 (1.83), 7.617 (1.72), 7.660 (4.22), 7.663 (4.25), 7.671 (2.96), 7.680 (2.91), 7.683 (2.63), 7.697 (2.70), 7.743 (3.06), 7.747 (3.14), 7.762 (2.60), 7.766 (2.44), 9.899 (6.83), 12.544 (0.41).

### ±

### Example 2

### Step 1: N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide (Compound XVII)

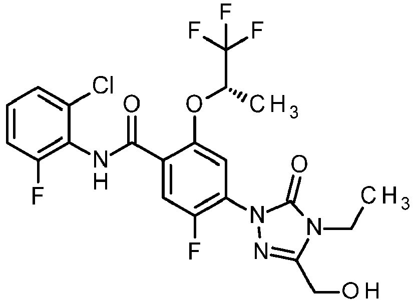

4-Bromo-N-(2-chloro-6-fluorophenyl)-5-fluoro-2-{[(2S)-1,1,1-trifluoropropan-2-yl]oxy}benzamide (Intermediate 7, 100 mg, 218 µmol), 4-ethyl-5-(hydroxymethyl)-2,4-dihydro-3H-1,2,4-triazol-3-one (Intermediate 8, 46.8 mg, 327 µmol), tris(dibenzylideneacetone)dipalladium(0) (20 mg, 22 µmol), 4,5-bis(diphenylphosphino)-9,9-dimethylxanthene (38 mg, 65 µmol), and cesium carbonate (142 mg, 436 µmol) were loaded into a microwave vial. The vial was purged with argon, dioxane (2 mL, degassed) was added, and the vial was sealed. The mixture was stirred for 17h at 110°C. The resulting suspension was filtered over Celite, washed with ethyl acetate and concentrated. Mass triggered preparative chromatography yielded the desired product (47.0 mg, 40 % yield).
LC-MS (Method A): Rₜ = 1.16 min; MS (ESIpos): m/z = 521 [M+H]⁺.
¹H-NMR (400 MHz, DMSO-d6) δ [ppm]: 1.137 (0.43), 1.233 (0.49), 1.264 (6.16), 1.282 (13.73), 1.300 (6.05), 1.436 (9.08), 1.451 (8.92), 2.084 (8.00), 2.322 (2.38), 2.326 (3.08), 2.331 (2.22), 2.518 (16.00), 2.522 (10.27), 2.664 (2.43), 2.669 (3.14), 2.673 (2.27), 3.764 (1.73), 3.782 (5.24), 3.800 (5.08), 3.818 (1.51), 4.479 (8.00), 4.493 (8.00), 5.331 (0.59), 5.347 (1.41), 5.363 (1.78), 5.380 (1.24), 5.396 (0.49), 5.759 (1.24), 5.789 (2.65), 5.803 (5.89), 5.817 (2.38), 7.337 (0.97), 7.356 (2.16), 7.372 (1.35), 7.380 (1.62), 7.388 (1.14), 7.408 (2.00), 7.422 (2.43), 7.433 (4.54), 7.439 (5.08), 7.453 (1.14), 7.550 (3.41), 7.575 (3.57), 7.588 (3.19), 7.602 (2.92), 10.065 (7.03).

### Experimental Design - Metabolite ID assay:

The *in vitro* metabolism of Example 2, Compound XVII was investigated time-dependently in hepatocytes of various species (i.e. human, mouse rat, dog and monkey). Freshly prepared hepatocytes were incubated in suspension with William's E medium (Sigma Aldrich) without FCS up to 4 h, using a cell density of 1x10⁶ cells/mL. The reactions were started by addition of 10µM Example 2 to the cell suspension and aliquots stopped at pre-determined time points (0, 1, 2 and 4h) by addition of acetonitrile and subsequent centrifugation of precipitated proteins. The supernatants were analyzed by HPLC-DAD-HRMS detection to generate metabolite profiles. Metabolite structures were elucidated by HPLC-MS/MS.

### Equipment:

QExactive™ Plus mass spectrometer
Orbitrap Fusion™ Lumos™ Tribrid™ mass spectrometer
Vanquish Horizon UHPLC System (Thermo Fisher Scientific, Waltham, MA, USA)
UPLC-system Acquity (Waters, Milford, MA, USA)

### Step 2: Metabolite 1

### N-(2-chloro-6-fluorophenyl)-5-fluoro-4-[3-(hydroxymethyl)-5-oxo-4,5-dihydro-1H-1,2,4-triazol-1-yl]-2-{[(2S)-1,1,1-trifluoropropan-2-yl]oxy}benzamide (Compound II)

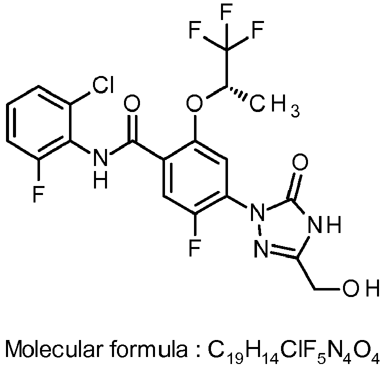

### Identification of metabolite 1:

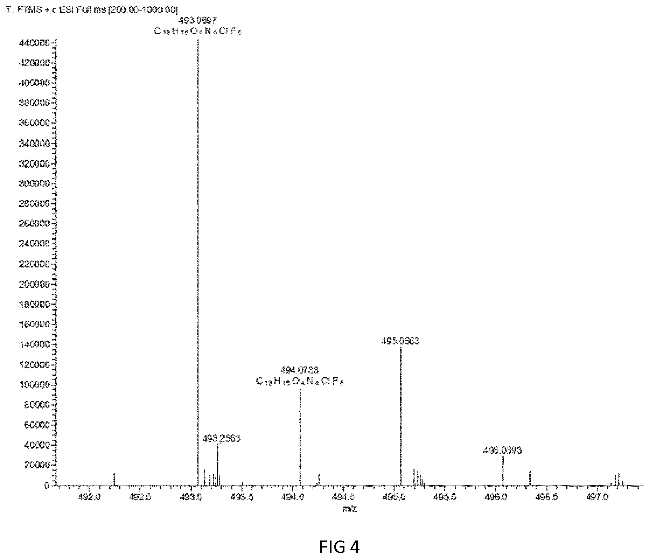

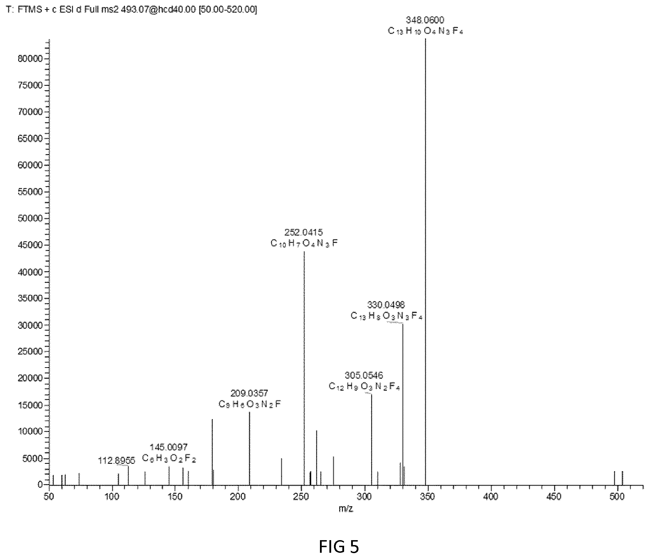

Metabolite 1 was identified in hepatocyte incubations with Compound (XVII) in vitro. A representative UV chromatogram (λ=300nm) at incubation start (see FIG 1) shows a peak at 7.46 min corresponding to Compound (XVII) 2 (MS: pseudomolecular ion [M+H]+ of m/z 521.1010). After 4h incubation time a decrease of the peak at 7.46 min and formation of several new peaks is observed, including one peak at 7.13 in (UV, see FIG 2) and 7.16 min (MS, see FIG 3) in the mass range of m/z 493.0671 to 493.0721. The high resolution mass spectrometric data at 7.16 min of the LC-MS/MS analysis showed a pseudomolecular ion [M+H]+ of m/z 493.0696 (see FIG 4), corresponding to a loss of 28Da compared to parent drug (Compound (XVII)) and a molecular formula of C19H15N4O4F5Cl. The MS2 product ion spectrum of m/z 493.07 showed major fragment ions of m/z 348.06 (loss of N-(chloro-fluorophenyl) moiety) and m/z 252.04 (loss of (chloro-fluorophenyl) moiety and trifluoropropyl substituent) (see FIG 5). Overall, the major and other minor fragments ions suggest that P-3 is an N-deethylated metabolite at the oxo-triazolyl region of Compound (XVII).

### Description of the figures:

**Fig1****:**
Representative UV Chromatogram (λ=300 nm) of Compound (XVII) incubated in hepatocytes at time point 0h (x-axis = Retention time [min]; y-axis = UV signal intensity [a.u.])

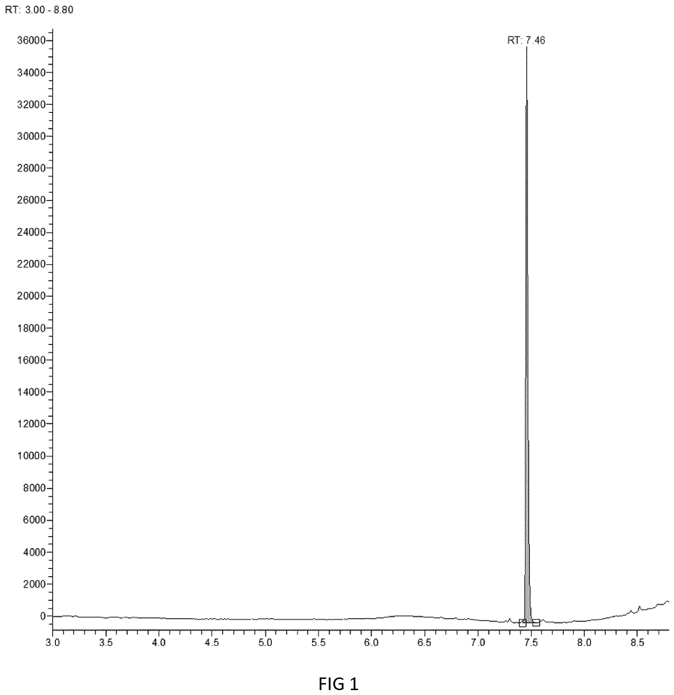

**Table 3 related to Figure 1:**

| **Retention time (Rt)** | **Peak Area (UV @ 300nm)** |
|---|---|
| [min] | [a.u.] |
| 7.46 | 48964 |

**FIG2****:**
Representative UV Chromatogram (λ=300 nm) of Compound (XVII) incubated in hepatocytes at time point 4h (x-axis = Retention time [min]; y-axis = UV signal intensity [a.u.])

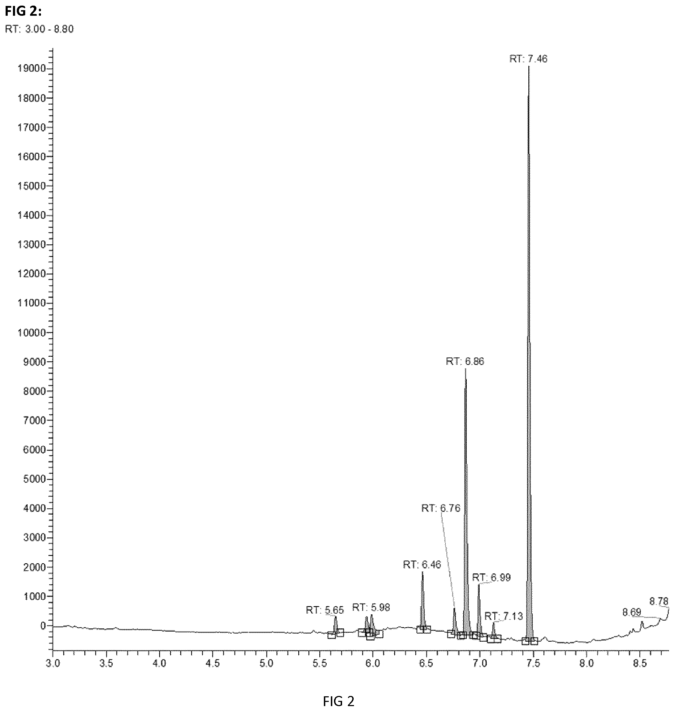

**Table 2 related to Figure 2:**

| **Retention time (Rt)** | **Peak Area (UV @ 300nm)** |
|---|---|
| [min] | [a.u.] |
| 5.65 | 1278 |
| 5.94 | 1342 |
| 5.98 | 1901 |
| 6.46 | 2831 |
| 6.76 | 1631 |
| 6.86 | 13687 |
| 6.99 | 2571 |
| 7.13 | 880 |
| 7.46 | 26078 |

**FIG3****:**
Representative MS chromatogram of selected mass range (m/z 493.0671 - 493.0721) of Compound (XVII) incubated in hepatocytes at time point 4h (x-axis = Retention time [min]; y-axis = MS signal intensity [a.u.]).

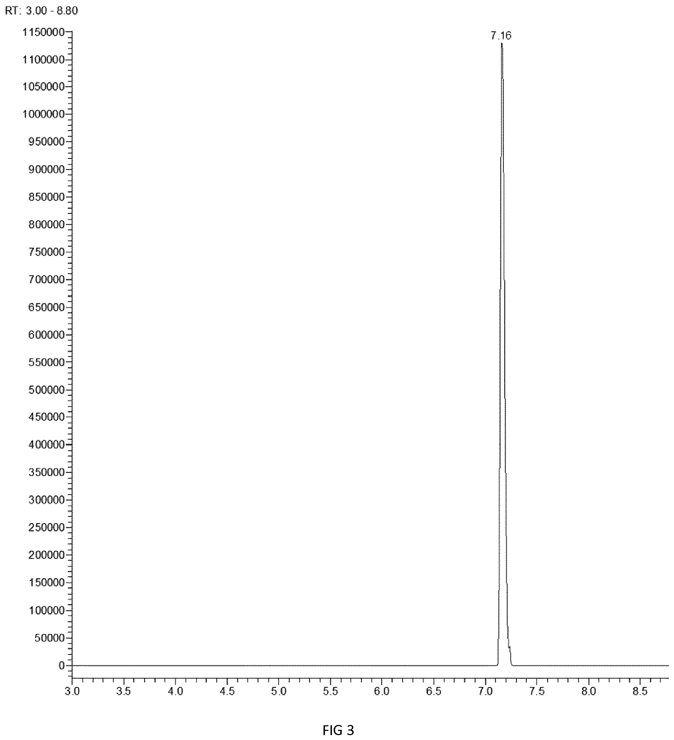

**Table 3 related to Figure 3:**

| **Retention time (Rt)** | **Signal Intensity (MS)** |
|---|---|
| [min] | [a.u.] |
| 7.16 | 1.13 x 10⁶ |

**FIG4****:**
Representative MS Parent Ion Spectrum of Compound (II) [M+H]⁺ (m/z = 493.0697) at Rt = 7.2min of the LC-MS analysis. Sample: Compound (XVII) incubation in hepatocytes at 4h time point (x-axis = m/z values; y-axis = MS signal intensity [a.u.]).
**FIG 5****:**
Representative MS² product ion spectrum of Compound (II) [M+H]⁺ (m/z = 493.0697) at Rt = 7.2min of the LC-MS/MS analysis. Sample: Compound (XVII) incubation in hepatocytes at 4h time point ((x-axis = m/z values; y-axis = MS signal intensity [a.u.]).

### EXPERIMENTAL SECTION - BIOLOGICAL ASSAYS

The following table 2 lists the abbreviations used herein, in particular in the Biological Assys part of the Experimental Section:

| | |
|---|---|
| ATCC | American Type Culture Collection |
| DDK | Name of a polypeptide tag |
| DCM | dichloromethane |
| DHODH | Dihydroorotate Dehydrogenase |
| DMSO | dimethylsulfoxide |
| h | hour(s) |
| IC₅₀ | half maximal inhibitory concentration |
| µM | micromolar |
| mM | millimolar |
| MTP | Microtiter plate |
| MYC | name of a polypeptide tag |
| µl | microliter |
| nM | nanomolar |
| PBS | Phosphate Buffered Saline |
| RPMI | Roswell Park Memorial Institute |
| rt | room temperature |
| THP | cell line name |
| Triton X | name of a detergent |
| Tris | tris(hydroxymethyl)aminomethane |

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assays:
***In vitro* Assay 1: DHODH enzymatic assay** - **1** The enzymatic assay couples DHODH activity with bleaching of the dye 2,6-dichlorophenolindophenol (DCIP) (Knecht and Loffler, 1998; Miller et al., 1968). The assay was conducted in buffer containing 50 mM Tris, 0.1% Triton X-100, 150 mM potassium chloride, 2 nM DHODH, 1 mM dihydroorotate, 0.1 mM decylubiquinone, 0.06 mM DCIP, and 2% DMSO at pH 8.0 at 32 degree celsius. The reaction was initiated by addition of substrates. Enzyme activity was monitored kinetically by the reduction in DCIP absorbance at 600 nm. Purified recombinant human DHODH enzyme was purchased from Novus (cat. no. NBP1-98916). Other chemicals were purchased from Sigma-Aldrich. Absorbance measurements were obtained using a BMG clarion star plate-reading spectrophotometer.

**Table 1: DHODH enzymatic assay : IC₅₀ values of examples in in vitro assay 1**

| **Compound** | **Target** |
|---|---|
| | **IC₅₀ [M/I]** |
| I | 3,00 E-6 |

***In vitro* Assay 2: THP-1 proliferation assay - 1 (AML)**
2000 cells/well of THP-1 cells were seeded in RPMI 1640 with Glutamax (Gibco, #11875-093) and 10% fetal calf serum (Biochrom, #S0615) in 384-well plates. The next day, cells were incubated with different concentrations of test compounds for 72h. Cellular viability was analyzed using CellTiter-Glo® Luminescent Cell Viability Assay (Promega, #G7570) according to manufacturer's instructions.

**Table 2: THP-1 proliferation assay:**

| **Compound** | **Target** |
|---|---|
| | **IC₅₀ [M]** |
| I | 3,05 E-7 |

## Claims

1. A compound (I) or (II): 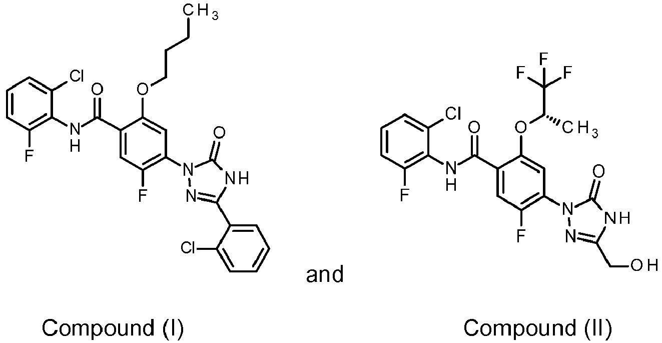 and stereoisomers, tautomers, N-oxides, hydrates, solvates, and salts thereof, and mixtures of same.

2. The compound (I) according to claim 1,2-butoxy-N-(2-chloro-6-fluorophenyl)-4-[3-(2-chlorophenyl)-5-oxo-4,5-dihydro-1H-1,2,4-triazol-1-yl]-5-fluorobenzamide, 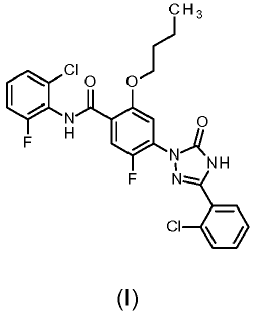 or a stereoisomer, a tautomer, an N-oxide, a hydrate, a solvate, or a salt thereof, or a mixture of same.

3. The compound (II) according to claim 1, N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, 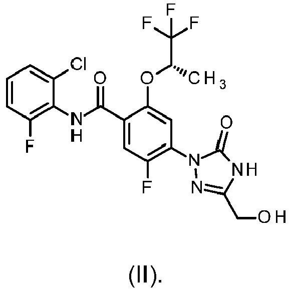 or a stereoisomer, a tautomer, an N-oxide, a hydrate, a solvate, or a salt thereof, or a mixture of same.

4. A method of preparing compound (I) according to calim 1 or 2, said method comprising the step of allowing an intermediate compound of formula (VIII) : 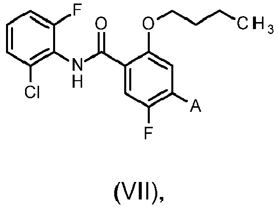 in which A is chlorine, bromine or iodine,
to react with a compound of formula (IX) : 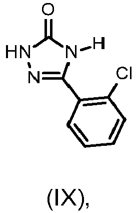 thereby giving a compound of formula (I) :

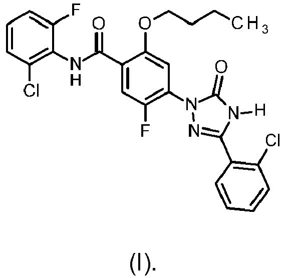

5. A method of preparing compound (II) according to calim 1 or 3, said method comprising the step of allowing an intermediate compound of formula (XVII) : 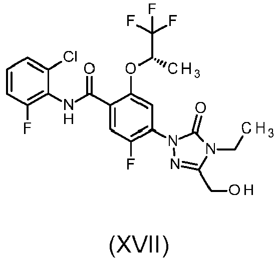 to be converted into 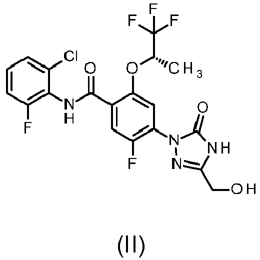 by a biological method.

6. The method according to claim 5, whereby the biological method comprises contacting compound (XVII) with hepatocytes, stopping the reaction by addition of an organic solvent and isolating the product by chromatography.

7. A compound (I) or (II) according to any one of claims 1 to 3 for use in the treatment or prophylaxis of a disease.

8. A compound (I) or (II) according to any one of claims 1 to 3 for use according to claim 8 wherein the disease is a hyperproliferative disorder.

9. A compound (I) or (II) according to any one of claims 1 to 3 for use according to claim 8 wherein the hyperproliferative disorders includes psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

10. A pharmaceutical composition comprising compound (I) and/or (II) according to any one of claims 1 to 3 and one or more pharmaceutically acceptable excipients.

11. A pharmaceutical combination comprising:
• one or more first active ingredients, in particular compounds (I) and/or (II) according to any one of claims 1 to 3, and

12. Use of compound (I) or (II) according to any one of claims 1 to 3 for the preparation of a medicament for the treatment or prophylaxis of a hyperproliferative disease, especially cancer.

13. Use according to claim 12, wherein the disease is a hyperproliferative disorder, such as a cancer disorder, for example the hyperproliferative disorder includes psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

14. An intermediate compound of geeral formula (II): 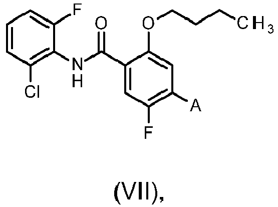 in which A is a chlorine, bromine or iodine atom, preferably a bromine atom.

15. Use of intermediate compound (VII) 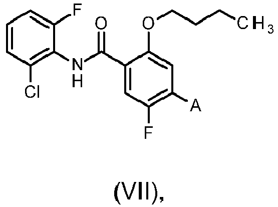 in which A is a chlorine, bromine or iodine atom, preferably a bromine atom,
for the preparation of compound (I) according to any one of claims 1 or 2.
